# EUROPEAN PATENT SPECIFICATION

(11) **EP 1 787 665 B1**
(45) Date of publication and mention of the grant of the patent: **15.07.2009**
(21) Application number: 06124497.6
(22) Date of filing: 21.11.2006
(51) Int. Cl.: A61M 1/16, A61M 16/00

(54) **System of modular integration of intravascular gas exchange catheter with respiratory monitor and ventilator**
System zur modularen Einpassung eines intravaskulären Gasaustauschkatheters mit Atemüberwachungsgerät und Beatmungsgerät
Système d'intégration modulaire d'un cathéter d'échange de gaz intravasculaire avec moniteur d'apnée et ventilateur

(30) Priority: 22.11.2005 US 284691
(43) Date of publication of application: 23.05.2007
(73) Proprietor: GENERAL ELECTRIC COMPANY, Schenectady, NY 12345 (US)
(72) Inventor: Barton, David F., McFarland, WI 53558 (US); Tham, Robert Q., Middleton, WI 53562 (US)
(74) Representative: Bedford, Grant Richard

(56) References cited:
- US-A- 4 850 958
- US-A- 5 219 326
- US-A- 5 336 164
- US-A1- 2004 052 681

## Description

The present invention relates to the field of patient ventilation. More specifically, the present invention relates to the field of artificial lung assist devices.

During intensive care therapy for patients with Chronic Obstructive Pulmonary Disease (COPD) and Acute Respiratory Distress Syndrome (ADRS), it is common for clinicians to utilize a respiratory carestation consisting of a critical care ventilator, a respiratory monitor and an information management system. The ventilator provides for the work of breathing based on the patient's clinical needs. The respiratory monitor allows the clinician to view patient waveforms, trends, gas monitoring including inspired and expired 02 and CO2 concentrations, End Tidal CO2 (ETCO2), CO2 production and 02 consumption, metabolics and energy expenditure, as well as patient spirometry. The information management system provides for patient data to be evaluated by the clinician either at the bedside or at a remote location.

An Intravascular Gas Exchange Catheter (IGEC), which in effect is an artificial lung assist device, consists of a multi-lumen catheter with a cylindrical bundle of microporous hollow fiber membranes woven into a mat at the end. The catheter is placed within the central venous blood stream in the primary vein that returns blood to the heart. Once inserted, oxygen gas flows from outside the patient, through the catheter and through the hollow fibers. As blood passes over the fibers, oxygen diffuses into the blood stream from the fibers, while carbon dioxide diffuses out of the blood stream into the fibers. Excess 02 and CO2 are removed back through the catheter out of the body. The device is inserted percutaneously via the femoral vein. A sutureless securement system with anti-microbial agents is then used to hold the catheter in place. The catheter fibers and components are coated with heparin to prevent coagulation.

Operation of Intravascular Gas Exchange Catheters has been discussed in prior-art literature. In particular, in U.S. Patent No. 4,850,958 (apparatus for extra-pulmonary blood gas exchange) and U.S. Patent No. 5,219,326 (inflatable percutaneous oxygenator). U.S. Patent No. 5,336,164 discloses an IGEC system comprising means for monitoring blood oxygenation. In other words, prior-art IGEC systems are essentially stand-alone devices that are controlled by an oxygenator.

Various aspects of the present invention relate to a system of integrating an intravascular gas exchange catheter with a patient respiratory system including a monitor and ventilator. The system obtains a monitoring sample of respiratory mechanic parameters for a present time interval, which may be selectively recurring over a predefined time. The system according to the aforementioned respiratory mechanic parameters, alerts a physician to adjust, or automatically adjusts the oxygen delivery through the IGEC, the ventilator operation, or both the IGEC and the ventilator.

A system of providing integrated care to a patient comprising a carestation configured to collect a monitoring sample of respiratory parameters, wherein the carestation determines whether the monitoring sample is within a predefined acceptable range, and an intravascular gas exchange catheter (IGEC) coupled to the carestation and inserted into the bloodstream of the patient, wherein the IGEC is adjustable when the monitoring sample is not within the predetermined range, wherein adjusting the IGEC controls an amount of oxygen that is added to the bloodstream of the patient and an amount of carbon dioxide removed from the bloodstream of the patient. The system further comprising an alarm means, wherein the alarm means is activated when the monitoring sample is not within the predetermined range, wherein the IGEC is adjusted manually by a user, and wherein the IGEC is adjusted automatically when the carestation sends an instruction signal to the IGEC, wherein the carestation includes a critical care ventilator, further wherein the critical care ventilator is adjustable when the monitoring sample is not within the predetermined range. The system, wherein the carestation collects the monitoring sample periodically when a user sets the carestation to an auto setting, wherein the IGEC is set to a starting level based on a set of patient physiological data, wherein the carestation is set to the predetermined acceptable range based on a set of patient physiological data, wherein the IGEC is inserted into the patient through the femoral vein, and wherein the carestation includes a critical care ventilator, a respiratory monitor and an information management system. The system, wherein the carestation is configured to monitor any of the following respiratory parameters: inspired and expired 02 and CO2 concentrations, end tidal CO2, CO2 production, 02 consumption, metabolics and energy expenditure, and patient spirometry.

Various aspects and embodiments of the present invention will now be described in connection with the accompanying drawings, in which:
Figure 1 illustrates a block diagram according to an embodiment of the present invention.
Figure 2 illustrates a flow chart depicting a method for use of an embodiment of the present invention.

The prior-art IGEC literature describes methods to operate an IGEC as a stand-alone device. However, the prior art does not describe the use of the IGEC in conjunction with a respiratory carestation including a ventilator, a respiratory monitor and an information management system. If used in conjunction with a respiratory carestation, the IGEC could provide the benefits of 02 and CO2 gas exchange while the ventilator is set on less aggressive settings. Significant side benefits for patients include reduced ventilator induced lung damage. It would also reduce the length of time to wean the critical care patient off the ventilator. The conjunction of the IGEC with the respiratory carestation could also significantly decrease the patient's ICU length of stay which improves patient quality of life and reduces cost to the healthcare system.

Recently, point of care therapy delivery devices (e.g. ICU ventilators) have further evolved to integrate more monitoring and therapy functions as well as serve as a bidirectional portal for the broader patient information network. These carestations integrate the activities of a variety of functions in using a common user interface and ergonomic physical function. On certain models, ventilation therapy is integrated with drug delivery through nebulizers, respiratory parameter measurements, respiratory gas monitoring, spirometry and metabolic monitoring. Similar carestations will integrate other types of physiologic monitoring as well, such as ECG, pulse oximetry and entropy. In addition, broadband communication capabilities to obtain information from electronic patient records such as pharmacy and lab data, for example blood gases, and digital imaging information.

One of the advantages of the carestation approach is that it allows the monitoring of therapy devices such as the IGEC so that the therapeutic benefits of the IGEC can be intimately linked to that of other therapy, for example ventilators and monitoring, patient gas monitoring, lung mechanics monitoring, and enhanced by the higher level of information present on the carestation. By integrating the monitoring of the IGEC in conjunction with this information, improved patient outcome can be obtained, especially for patients suffering from COPD and ARDS.

Specifically, in one aspect, the present invention relates to a respiratory therapy carestation, defined as the combination of at least a ventilation delivery device (ventilator) and IGEC. The carestation has a fundamental ability to evaluate the oxygenation level of a patient's blood and provides the clinician with that information during the time that the patient is mechanically ventilated. With this ability the carestation is claimed to provide optimization of oxygen delivery and carbon dioxide removal.

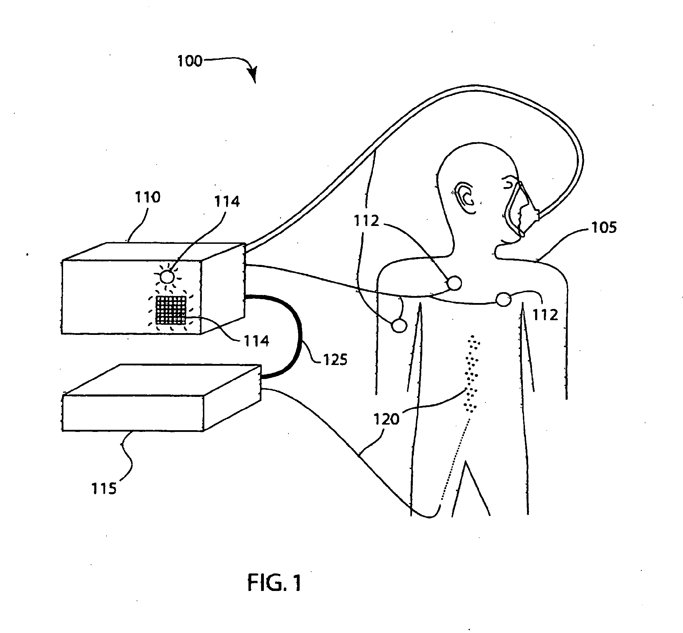

The respiratory carestation system 100 of an embodiment of the present invention is depicted in Figure 1. In Figure 1, a patient 105 is monitored by a carestation 110, utilizing a number of physiological sensors 112, as required to collect the various physiological parameters set as patient waveforms, trends, gas monitoring, including inspired and expired O2 and CO2 concentrations, end title CO2 (ETCO2), CO2 production and O2 consumption, metabolic and energy expenditure, as well as patient spirometry. The carestation 110 collects this information from the patient 105 and compares it to an acceptable predetermined and preset range. If the physiological parameters of the patient 105 are not within that predetermined, preset range, the alarming means 114 of the carestation 112 will alert a user of the respiratory carestation system 100 of such a condition. The alarming means 114 may be visual, such as a light, and/or an audible alarm. The alarming means 114 will alert a user of the respiratory carestation system 100, so that the user may adjust the IGEC control 115 accordingly, so that the patient 105 may receive the appropriate amount of blood oxygenation from the IGEC 120. The respiratory system 100 is also configured such that the user may adjust the ventilator in the carstation 110, or a combination of the ventilator and the IGEC 120 in order to return the patient's 105 physiological parameters to the acceptable range.

Still referring to Figure 1, the IGEC 120 is preferably inserted through the femoral vein of the patient 105, and operates as described above. The IGEC 120 is controlled by an IGEC control 115, and is coupled through an IGEC coupling 125 to the carestation 110. In additional embodiments of the present invention, when the patient 105 is displaying parameters that are outside the predetermined, preset range, the carestation 110 will detect this condition, and instruct the IGEC control 115 through the IGEC coupling 125 to adjust the oxygenation through the IGEC 120 automatically, and as described previously, the system 100 will be configured to adjust the ventilator automatically, as well as the ventilator and IGEC 120 in combination in order to return the patients 105 physiological parameters back to an acceptable range.

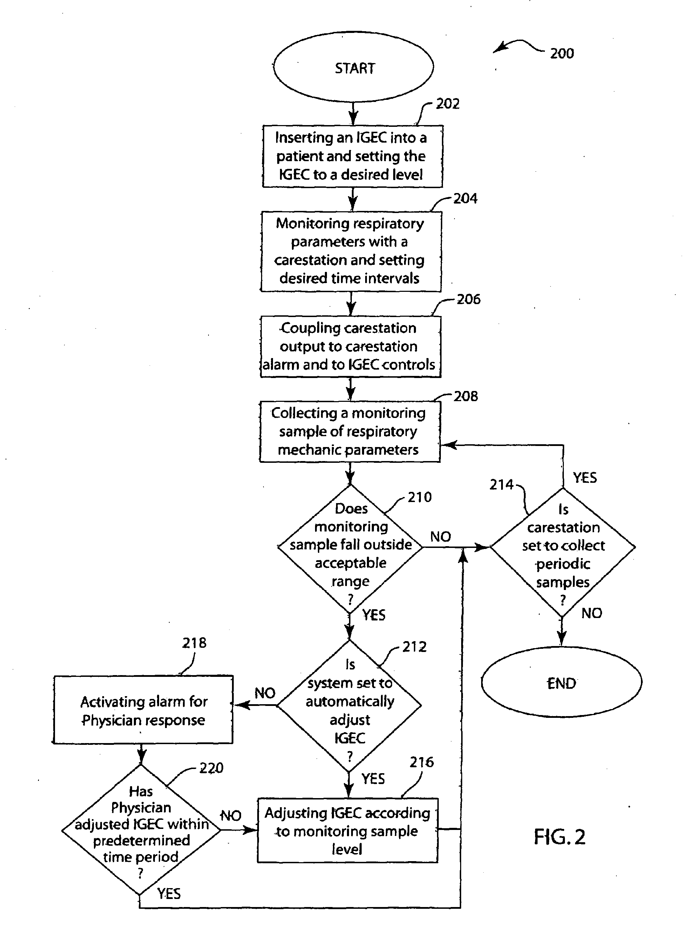

Referring now to Figure 2, an integration method 200 for use of an embodiment of the present invention is depicted. In step 202, an IGEC is inserted into a patient and set to a desired oxygenation level. In step 204, the respiratory parameters are monitored with the carestation and desired time levels of sampling these respiratory parameters are set. In step 206, the carestation output is coupled to a carestation alarm, and to the IGEC controls.

Still referring to Figure 2, in step 208, a monitoring sample of respiratory mechanic parameters are collected by the carestation. In step 210 it is determined whether the monitoring sample falls outside the acceptable range. If the monitoring does not fall outside the acceptable range in step 210, in step 214, it is determined whether the carestation is set to collect periodic samples. If the carestation is so set, then a new monitoring sample is collected in step 208. If the carestation is not set to collect periodic samples, then the integration method ends. Referring back to step 210, if the monitoring sample does fall outside the acceptable range then, in step 212, it is determined whether the system is set to automatically adjust the IGEC. If the system is set to automatically adjust the IGEC, then in step 216, the IGEC is adjusted according to the monitoring sample level, and the integration method 200 continues onto step 214, which is described earlier in this description. Referring back to step 216, the method is also configured such that the ventilator is adjusted in combination with the IGEC.

If the system is not set to automatically adjust the IGEC, then in step 218, an alarm is activated for physician response. In step 220, if the physician has adjusted the IGEC within a predetermined time period, then the integration method 200 continues onto step 214, which is described above. If the physician does not adjust the IGEC within the predetermined time period, then in step 216, the IGEC is automatically adjusted according to the monitoring sample level. In step 220, the physician may also adjust the ventilator in combination with the IGEC.

The respiratory carestation's integration with IGEC delivery allows delivery of respiratory therapy in a more optimized fashion than can be accomplished with prior art systems. Further, when the respiratory carestation includes respiratory mechanics monitoring, automatic assessments of the patient respiratory condition can be accomplished and linked to appropriately aggressive use of the respiratory ventilator.

With the introduction of the integrated respiratory carestations described in this invention, a patient's level of blood oxygenation can be further optimized based on information obtained or generated by the ventilator or respiratory mechanics monitoring system. The respiratory carestation including respiratory mechanics monitoring offers the ability to automatically assess the effectiveness of combined therapy of IGEC and respiratory ventilator (and even control the delivery of such therapy in a closed loop fashion based on the respiratory mechanics monitoring results).

The present invention has been described in terms of specific embodiments incorporating details to facilitate the understanding of the principles of construction and operation of the invention. Such reference herein to specific embodiments and details thereof is not intended to limit the scope of the claims appended hereto. It will be apparent to those skilled in the art that modifications may be made in the embodiment chosen for illustration without departing from the scope of the invention.

**PARTS LIST**

| **Component Part** | **Reference Number** | **Figure Number** |
|---|---|---|
| Respiratory Carestation System | 100 | Fig. 1 |
| Patient | 105 | Fig. 1 |
| Carestation | 110 | Fig. 1 |
| Physiological Sensors | 112 | Fig. 1 |
| Alarming Means | 114 | Fig. 1 |
| IGEC Control | 115 | Fig. 1 |
| IGEC | 120 | Fig. 1 |
| Integration Method | 200 | Fig. 2 |
| Step | 202 | Fig. 2 |
| Step | 204 | Fig. 2 |
| Step | 206 | Fig. 2 |
| Step | 208 | Fig. 2 |
| Decision | 210 | Fig. 2 |
| Decision | 212 | Fig. 2 |
| Decision | 214 | Fig. 2 |
| Step | 216 | Fig. 2 |
| Step | 218 | Fig. 2 |
| Decision | 220 | Fig. 2 |
| IGEC Coupling | 125 | Fig. 1 |
| | | |

## Claims

1. A system (100) for providing integrated care to a patient (105), the system comprising:
a.) a carestation (110) configured to collect a monitoring sample of respiratory parameters, wherein the carestation determines whether the monitoring sample is within a predefined acceptable range; and
b.) an intravascular gas exchange catheter (IGEC) (120) coupled to the carestation and for inserting into the bloodstream of the patient, wherein the IGEC is adjustable when the monitoring sample is not within the predetermined range,
wherein adjusting the IGEC (120) controls an amount of oxygen that is added to the bloodstream of the patient and an amount of carbon dioxide removed from the bloodstream of the patient

2. The system (100) of claim 1, further comprising an alarm means (114), wherein the alarm means is activated when the monitoring sample is not within the predetermined range.

3. The system (100) of claim 1 or claim 2, wherein the IGEC (120) is adjusted manually by a user.

4. The system (100) of any preceding claim, wherein the IGEC (120) is adjusted automatically when the carestation sends an instruction signal to the IGEC.

5. The system (100) of any preceding claim, wherein the carestation (110) includes a critical care ventilator, further wherein the critical care ventilator is adjustable when the monitoring sample is not within the predetermined range.

6. The system (100) of any preceding claim, wherein the carestation collects the monitoring sample periodically when a user sets the carestation to an auto setting.

7. The system (100) of any preceding claim, wherein the IGEC (120) is set to a starting level based on a set of patient physiological data.

8. The system (100) of any preceding claim, wherein the carestation (110) is set to the predetermined acceptable range based on a set of patient physiological data.

9. The system (100) of any preceding claim, wherein the IGEC (120) is insertable into the patient through the femoral vein.

10. The system (100) of any preceding claim, wherein the carestation (110) includes a critical care ventilator, a respiratory monitor and an information management system.

## Patentansprüche

1. System (100) zur Bereitstellung einer integrierten Behandlung für einen Patienten (105), wobei das System aufweist:
a) eine Behandlungsstation (110), die dafür konfiguriert ist, eine Überwachungsstichprobe von Atmungsparametern zu sammeln, wobei die Behandlungsstation ermittelt, ob sich die Überwachungsstichprobe innerhalb eines vorbestimmten zulässigen Bereiches befindet; und
b) einen mit der Behandlungsstation verbundenen und für die Einführung in den Blutstrom des Patienten gedachten intravaskulären Gasaustauschkatheter (IGEC - Intravascular Gas Exchange Catheter) (120), wobei der IGEC einstellbar ist, wenn die sich Überwachungsstichprobe nicht innerhalb des vorbestimmten Bereiches befindet,
wobei das Einstellen des IGEC (120) eine dem Blutstrom des Patienten zugesetzte Sauerstoffmenge und eine Menge des aus dem Blutstrom des Patienten abgeführten Kohlendioxids steuert.

2. System (100) nach Anspruch 1, welches ferner eine Alarmeinrichtung (114) aufweist, wobei die Alarmeinrichtung aktiviert wird, wenn sich die Überwachungsstichprobe nicht innerhalb des vorbestimmten Bereiches befindet.

3. System (100) nach Anspruch 1 oder Anspruch 2, wobei der IGEC (120) manuell durch einen Benutzer eingestellt wird.

4. System (100) nach einem der vorstehenden Ansprüche,
wobei der IGEC (120) automatisch eingestellt wird, wenn die Behandlungsstation ein Anweisungssignal an den IGEC sendet.

5. System (100) nach einem der vorstehenden Ansprüche,
wobei die Behandlungsstation (110) eine Intensivbeatmungseinrichtung enthält, wobei ferner die Intensivbeatmungseinrichtung einstellbar ist, wenn sich die Überwachungsstichprobe nicht innerhalb des vorbestimmten Bereiches befindet.

6. System (100) nach einem der vorstehenden Ansprüche,
wobei die Behandlungsstation die Überwachungsstichprobe periodisch sammelt, wenn ein Benutzer die Behandlungsstation auf einen automatischen Betrieb einstellt.

7. System (100) nach einem der vorstehenden Ansprüche,
wobei der IGEC (120) auf der Basis eines Satzes physiologischer Daten des Patienten auf einen Ausgangswert eingestellt wird.

8. System (100) nach einem der vorstehenden Ansprüche,
wobei die Behandlungsstation (110) auf der Basis eines Satzes physiologischer Daten des Patienten auf den vorbestimmten zulässigen Bereich eingestellt wird.

9. System (100) nach einem der vorstehenden Ansprüche,
wobei der IGEC (120) in den Patienten über eine Oberschenkelvene einführbar ist.

10. System (100) nach einem der vorstehenden Ansprüche,
wobei die Behandlungsstation (110) eine Intensivbeatmungseinrichtung, eine Atmungsüberwachungseinrichtung und ein Informationsmanagementsystem enthält.

## Revendications

1. Système (100) pour dispenser des soins intégrés à un patient (105), le système comprenant :
a) une station de soins (110) configurée pour collecter un échantillon de surveillance de paramètres respiratoires, dans lequel la station de soins détermine si l'échantillon de surveillance se trouve dans une plage acceptable prédéfinie ; et
b) un cathéter d'échange gazeux intravasculaire (CEGI) (120) couplé à la station de soins et destiné à être inséré dans le courant sanguin du patient, dans lequel le CEGI est réglable lorsque l'échantillon de surveillance ne se trouve pas dans la plage prédéterminée,
dans lequel le réglage du CEGI (120) contrôle une quantité d'oxygène qui est ajoutée au courant sanguin du patient et une quantité de gaz carbonique éliminée du courant sanguin du patient.

2. Système (100) selon la revendication 1, comprenant en outre un moyen d'alarme (114), dans lequel le moyen d'alarme est activé lorsque l'échantillon de surveillance ne se trouve pas dans la plage prédéterminée.

3. Système (100) selon la revendication 1 ou la revendication 2, dans lequel le CEGI (120) est réglé manuellement par un utilisateur.

4. Système (100) selon l'une quelconque des revendications précédentes, dans lequel le CEGI (120) est réglé automatiquement lorsque la station de soins envoie un signal d'instruction au CEGI.

5. Système (100) selon l'une quelconque des revendications précédentes, dans lequel la station de soins (110) comprend un ventilateur pour malades en phase critique, dans lequel en outre le ventilateur pour malades en phase critique est réglable lorsque l'échantillon de surveillance ne se trouve pas dans la plage prédéterminée.

6. Système (100) selon l'une quelconque des revendications précédentes, dans lequel la station de soins collecte l'échantillon de surveillance périodiquement lorsqu'un utilisateur règle la station de soins sur un paramètre automatique.

7. Système (100) selon l'une quelconque des revendications précédentes, dans lequel le CEGI (120) est réglé sur un niveau de départ basé sur un ensemble de données physiologiques du patient.

8. Système (100) selon l'une quelconque des revendications précédentes, dans lequel la station de soins (110) est réglée sur la plage acceptable prédéterminée basée sur un ensemble de données physiologiques du patient.

9. Système (100) selon l'une quelconque des revendications précédentes, dans lequel le CEGI (120) peut être inséré dans le corps du patient par la veine fémorale.

10. Système (100) selon l'une quelconque des revendications précédentes, dans lequel la station de soins (110) comprend un ventilateur pour malades en phase critique, un moniteur de respiration et un système de gestion des informations.
